(19) Europäisches Patentamt
European Patent Office
Office européen des brevets

(11) Publication number: **0 007 736**
**B1**

(12) # EUROPEAN PATENT SPECIFICATION

(45) Date of publication of patent specification: **12.10.83**

(51) Int. Cl.³: **C 08 L 23/00**, C 08 K 5/34, A 61 L 2/08

(21) Application number: **79301347.5**

(22) Date of filing: **10.07.79**

(54) **Polyolefin articles sterilisable by gamma-irradiation.**

(30) Priority: **25.07.78 GB 3108678**

(43) Date of publication of application:
**06.02.80 Bulletin 80/3**

(45) Publication of the grant of the patent:
**12.10.83 Bulletin 83/41**

(84) Designated Contracting States:
**AT BE CH DE FR GB IT LU NL SE**

(56) References cited:
CH - A - 586 252
CH - A - 597 297
CH - A - 597 298
DE - B - 1 929 928
DE - B - 2 204 659

(73) Proprietor: **IMPERIAL CHEMICAL INDUSTRIES PLC**
**Imperial Chemical House Millbank**
**London SW1P 3JF (GB)**

(72) Inventor: **Rayner, Martin Geoffrey**
**8 Valley Road**
**GB-Codicote Hertfordshire (GB)**
Inventor: **Harris, Colin Andrew**
**17 Stoneycroft**
**Welwyn Garden City Hertfordshire AL7 1QU (GB)**

(74) Representative: **Rhind, John Lessels et al,**
**Imperial Chemical Industries PLC Legal Department: Patents Thames House North Millbank**
**London SW1P 4QG (GB)**

EP 0 007 736 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Polyolefin articles sterilisable by gamma-irradiation

This invention relates to polyolefin articles, especially polypropylene hypodermic syringe components which are suitable for sterilisation by gamma-irradiation from a cobalt-60 source.

Polypropylene syringes have been in use for many years (see British Patent Specification 1 050 802 published in 1966) and have been sterilised by gamma-irradiation even though this causes yellow discolouration and/or embrittlement of the syringe. Plester reports (see "Bio-Medical Engineering" September 1970, pages 443 to 447) that polypropylene is of only "borderline" suitability for such use and accordingly many attempts have been made to alleviate the problem. Nevertheless, the problem has persisted and was discussed and left unsolved by Williams et al in "Radiation Physics and Chemistry", Volume 9, pages 445 to 454, published in 1977.

It has now been discovered that polyolefins containing certain hindered amines are more resistant to the discolouration which occurs as a result of gamma-irradiation. Typical hindered amines are described in West German Patent Specifications 2 204 659 and 2 319 816, published in 1972 and 1973 respectively.

Accordingly, this invention provides an article of medical ware comprising a syringe component, catheter, cannula or prosthetic device, said article being sterilisable by gamma-irradiation from a cobalt-60 source and made from a composition comprising a crystalline polymer of an aliphatic-olefin characterised in that the composition comprises from 0.01 to 2 (preferably 0.08 to 0.3%) by weight of the polymer of a hindered amine or its salt, N-oxide, N-hydroxide or N-nitroxide wherein the amine nitrogen is contained in a carbon-nitrogen-carbon chain which forms part of a non-aromatic heterocyclic ring and wherein each of the two carbon atoms of the chain is bonded to two same or different lower alkyl groups each containing 1 to 12 carbon atoms or to an alicyclic group containing 4 to 9 carbon atoms which groups sterically hinder the amine.

The invention also provides a method of forming a sterilised article of medical ware comprising a syringe component, catheter, cannula or prosthetic device characterised by forming the article from a composition as immediately hereinbefore defined and exposing the formed article to a sterilising dose of gamma-irradiation from a cobalt-60 source.

In general, the articles can receive a radiation dose of 5.0 Mrad from a cobalt-60 source and then have a discolouration factor (as hereinafter defined) of better than 0.750 (often better than 0.800) two weeks after irradiation and an oven life in air at 150°C of at least 6 and often over 10 days in sections 3.17 mm thick.

The hindered amines preferably comprise a 5- or 6-membered heterocyclic ring containing the hindered amine nitrogen and optionally another hetero atom preferably nitrogen or oxygen. If the hindered amine is a tertiary amine, the tertiary group may be, for example, an optionally substituted alkyl, aralkyl, alkaryl or alicyclic group containing 1 to 12 carbon atoms and one or more of the substituents may be hindered amines so that the tertiary group may be used to link a plurality of hindered amines. The hindering groups are preferably alkyl groups containing 1 to 4 carbon atoms and most preferably all four groups are methyl. The most preferred hindered amines comprise 2,2,4,4-tetramethyl piperidine derivatives.

The hindered amine is preferably bonded to a carrier moiety which should have little if any inhibiting effect on the chemical activity of the hindered amine. Reasonably inert carriers include aromatic compounds (for example those based on the benzene, imidazole or triazine rings), saturated hydrocarbon compounds, esters or amides of carboxylic acids, ketones and ether, thioether, sulphinyl or sulphone groups. Preferably, in order to reduce the extractability of the hindered amines from polyolefins, the carriers can be used to link together a plurality of hindered amines and hence the tertiary groups may be regarded as carriers too.

Examples of hindered amines linked by dicarboxylate or diketone moieties include:

a) di-(2,2,6,6-tetramethyl-4-piperidyl) sebacate (see formula I) where it is the sebacate which is the carrier,

b) di-(2,2,6,6-tetramethyl-4-piperidyl) 1-(3,5-ditertiarylbutyl-4-hydroxyphenylmethyl)-1,1-pentanedicarboxylic (see formula II) where it is the pentanedicarboxylate which is the carrier.

c) the condensate of succinic acid and N-(2-hydroxypropyl)-2,2,6,6-tetramethyl-4-hydroxy-piperidine (see formula III) where both the succinate moiety and the tertiary propoxy groups combine to form carriers. The condensate preferably contains 6 to 20 hindered amine groups, and

d) 1,4-di-(2,2,6,6-tetramethyl-4-piperidyl)-2,3-butanedione (see formula IV) where it is the butanedione which is the carrier.

The polyolefin can be, for example, a homopolymer of ethylene (low or high density polyethylene), propylene, butene-1 or 4-methyl-pentene-1 or a copolymer of two or more of these monomers. Preferred copolymers are copolymers of propylene and either from 7 to 20% (by weight of the copolymer) of ethylene when made by injecting ethylene into the latter stages of an otherwise homopolymerisation of propylene or from 0.5 to 10% of ethylene when made by random copolymerisation. Copolymers of ethylene with up to 30% of vinyl acetate, methyl, ethyl or butyl

(including tertiary butyl) acrylates or methacrylates or acrylic or methacrylic acids may also be used. Preferably, the polyolefins have melt flow indexes of from 0.1 to 25 g/10 minutes when measured according to British Standard 2782 Part 1/105C of 1970 using a 2.16 kg load at 230°C in the case of polymers containing a major amount of polymerised propylene and at 190°C in all other cases. Added rubbers improve impact strength.

The composition may contain stabilising amounts (e.g. 0.01 to 2% by weight of the polymer) of light stabilisers (e.g. benzotriazoles) and/or phenolic antioxidants of the kind used in polyolefins, for example, n-octadecyl 3-(3,5-ditertiarybutyl-4-hydroxyphenyl) propionate. However, phenolic antioxidants may aggravate discolouration so their use may entail sacrifice of some of the improvement in discolouration. The compositions may also contain stabilising amounts of sulphur compounds of the type known to synergise with phenolic antioxidants in polyolefins, for example, long chain (e.g. $C_{10}$ to $C_{22}$) mercaptans and sulphides but the preferred compounds are dialkyl thiodialkanoates especially when the alkyl groups contain from 10 to 22 carbon atoms and the alkanoic acids contain from 2 to 6 carbon atoms. Specific examples are dilauryl and distearyl thiodipropionate. The compositions preferably contain from 0.01 to 10% (usually 0.1 to 0.5%) by weight of organic sulphur compound. Other conventional additives such as pigments or moulding aids may be used.

Articles of sterilisable medical ware according to this invention comprise syringe components (such as barrels, plungers and needle hubs), catheters, cannulae and prosthetic devices.

The invention is illustrated by the following examples of which A to I are comparative.

Examples A to I and 1 to 3

Using a high speed powder mixer, powdered propylene homopolymer having a melt flow index of 8 g/10 minutes was mixed with 0.2% by weight of powdered calcium stearate and various amounts of various powdered additives as specified in Table 1. (The percentages specified in Table 1 and of calcium stearate are based on the weight of the polymer). Each mixture of powders was heated to 225°C to melt the polymer and the melt was converted to granules. Each batch of granules was injection moulded into discs 3.17 mm thick and 11.4 cm in diameter.

Sample discs were retained as controls and the rest were irradiated at 2.5 or 5.0 Mrad using a cobalt-60 source. The irradiated discs were divided into three groups. The first group were kept under ambient conditions for 2 weeks and then their discoloration factors were measured. They were then kept under ambient conditions for a period of three months after irradiation and then their discolouration factors were measured again.

The second group of discs were kept under ambient conditions for 4 weeks and then placed in an air-circulating oven maintained at 100°C for three days. On removal from the oven and cooling to ambient temperature, the discolouration factors of the discs were measured.

The third group of discs were put into an air-circulating oven maintained at 150°C and the time taken for them to embrittle (i.e. for surface cracking to appear) was noted. All the results are shown in Table 2.

"Discolouration factor" is measured using a 'Colormaster' differential colorimeter as supplied by the Manufacturers, Engineering and Equipment Corporation of Warrington, Pennsylvania, U.S.A.

A beam of blue light from the 'Colormaster' was shone onto a white surface at an angle of incidence of 45° so as to illuminate an area of the surface. The intensity of light leaving the illuminated area in a direction normal to the surface was registered by the 'Colormaster'. A disc 3.17 mm thick was then placed over the illuminated area and was compared by the 'Colormaster'. The procedure was repeated using red light and the ratio of loss of intensity of blue light to loss of intensity of red light as compared by the 'Colormaster' was calculated. This ratio is defined as the "discolouration factor".

The higher the discolouration factor, the lower is the yellow discolouration of the sample. Therefore, it will be seen that in general the least yellow discolouration occurred in Examples 1, 2, 3 and A, but in the case of A, the brittleness of the sample was unacceptable.

TABLE 1

| Example | % by weight antioxidant | % by weight thioester | % by weight other *additive |
|---|---|---|---|
| A | — | — | — |
| B | — | 0.3 | — |
| C | 0.1 | 0.3 | — |
| D | 0.2 | 0.5 | — |
| E | 0.1 | 0.3 | 0.3a |
| F | 0.1 | 0.3 | 0.1b |
| G | 0.1 | 0.3 | 0.1c |
| H | 0.1 | 0.3 | 0.1d |
| I | Proprietry Composition | | |
| 1 | 0.1 | — | 0.2e |
| 2 | — | — | 0.2f |
| 3 | 0.1 | — | 0.2g |

*Additives

a = di-benzylidene sorbitol.
b = a mixture of tri-mono and tri-de-nonyl phenyl phosphite containing 1% by weight (of the weight of the phosphite) of triisopropanol amine.
c = tris-(2,4-ditertiary butyl phenyl) phosphite.
d = a proprietry organic phosphonite.
e = as formula III.
f = as formula II.
g = as formula I.

TABLE 2

| Ex. | Irradiation dose Megarads | | | | | | | | |
|---|---|---|---|---|---|---|---|---|---|
| | 0 | | 2.5 | | | | 5.0 | | |
| | | | Discolouration factor | | | | Discolouration factor | | |
| | Discolouration factor | Oven life days | After 2 weeks | After 4 weeks + 3 days at 100°C | 3 months | Oven life days | After 2 weeks | After 4 weeks + 3 days at 100°C | Oven life days |
| A | 0.941 | 3 | 0.940 | Brittle | — | ⩽3 | 0.847 | Brittle | ⩽3 |
| B | 0.949 | 6 | 0.918 | 0.473 | — | ⩽3 | 0.713 | 0.488 | ⩽3 |
| C | 0.940 | 49 | 0.813 | 0.740 | — | 17 | 0.679 | 0.593 | ⩽3 |
| D | 0.927 | — | 0.766 | 0.730 | — | 31 | 0.593 | 0.535 | ⩽3 |
| E | 0.935 | 54 | 0.786 | 0.737 | — | 21 | 0.648 | 0.555 | ⩽3 |
| F | 0.913 | 46 | 0.760 | 0.691 | — | 18 | 0.596 | 0.546 | ⩽3 |
| G | 0.939 | — | 0.798 | 0.762 | — | 19 | 0.630 | 0.562 | ⩽3 |
| H | 0.944 | — | 0.798 | 0.742 | — | 18 | 0.672 | 0.581 | ⩽3 |
| I | 0.890 | 59 | 0.772 | 0.707 | — | 3 | 0.634 | 0.584 | ⩽3 |

TABLE 2 (continued)

| | Irradiation dose Megarads | | | | | | | | |
|---|---|---|---|---|---|---|---|---|---|
| | 0 | | 2.5 | | | | 5.0 | | |
| | | | Discolouration factor | | | | Discolouration factor | | |
| Ex. | Discolouration factor | Oven life days | After 2 weeks | After 4 weeks + 3 days at 100°C | 3 months | Oven life days | After 2 weeks | After 4 weeks + 3 days at 100°C | Oven life days |
| 1 | 0.923 | 33 | 0.886 | 0.802 | 0.907 | 25 | 0.870 | 0.741 | 12 |
| 2 | 0.899 | 31 | 0.855 | 0.786 | 0.900 | 25 | 0.827 | 0.736 | 19 |
| 3 | 0.931 | 19 | 0.829 | 0.686 | 0.885 | 25 | 0.830 | 0.568 | >20 |

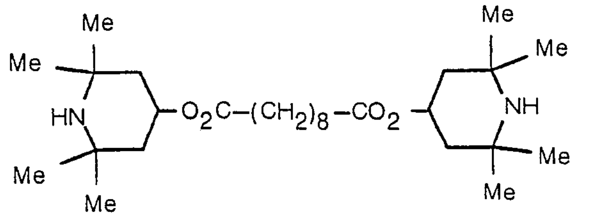

I

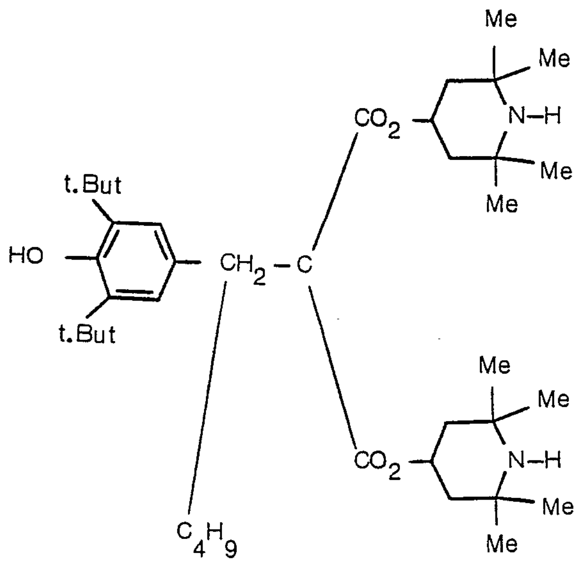

II

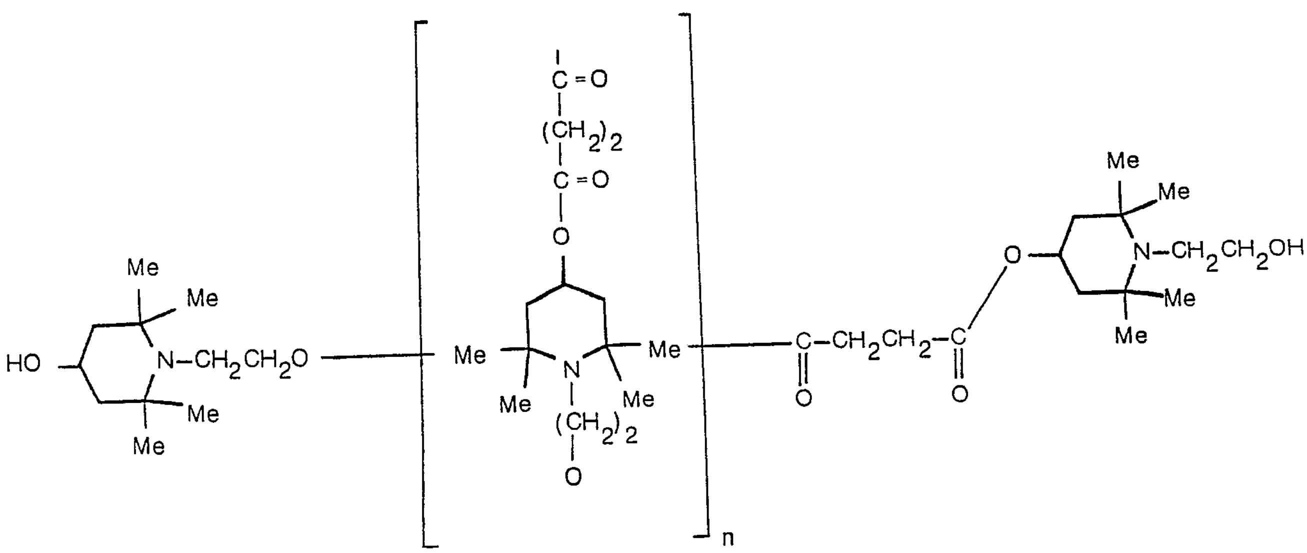

III

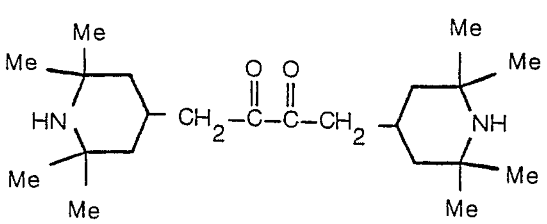

IV

**Claims**

1. An article of medical ware comprising a syringe component, catheter, cannula or prosthetic device, said article being sterilisable by gamma-irradiation from a cobalt-60 source and made from a composition comprising a crystalline polymer of an aliphatic alpha-olefin characterised in that the composition comprises from 0.01 to 2% by weight of the polymer of a hindered amine or its salt, N-oxide, N-hydroxide or N-nitroxide wherein the amine nitrogen is contained in a carbon-nitrogen-carbon chain which forms part of a non-aromatic heterocyclic ring and wherein each of the two carbon atoms of the chain is bonded to two same or different lower alkyl groups each containing 1 to 12 carbon atoms or to an alicyclic group containing 4 to 9 carbon atoms which groups sterically hinder the amine.

2. An article as claimed in claim 1 wherein the hindered amine comprises a 6-membered heterocyclic ring.

3. An article as claimed in claim 2 wherein the hindered amine is carried by a carrier comprising a dicarboxylate.

4. An article as claimed in claim 2 wherein the hindered amine is carried by a carrier comprising a triazine ring.

5. An article as claimed in claim 2 wherein the hindered amine is carried by a carrier comprising a diketone.

6. An article of medical ware comprising a syringe component, catheter, cannula or prosthetic device sterilised by gamma-irradiation from a cobalt-60 source and made from a composition comprising a crystalline polymer of an aliphatic alpha-olefin wherein the composition comprises from 0.01 to 2% by weight of the polymer of a hindered amine or its salt, N-oxide, N-hydroxide or N-nitroxide wherein the amine nitrogen is contained in a carbon-nitrogen-carbon chain which forms part of a non-aromatic heterocyclic ring and wherein each of the two carbon atoms of the chain is bonded to two same or different lower alkyl groups each containing 1 to 12 carbon atoms or to an alicyclic group containing 4 to 9 carbon atoms which groups sterically hinder the amine.

7. An article according to claim 6 having a discolouration factor of better than 0.750 two weeks after irradiation and an oven life in air at 150°C of at least 6 days, said article having received a radiation dose of 5.0 Mrad from a cobalt-60 source.

8. A method of forming a sterilised article of medical ware comprising a syringe component, catheter, cannula or prosthetic device characterised by forming the article from a composition comprising from 0.01 to 2% by weight of the polymer of a hindered amine or its salt, N-oxide, N-hydroxide or N-nitroxide wherein the amine nitrogen is contained in a carbon-nitrogen-carbon chain which forms part of a non-aromatic heterocyclic ring and wherein each of the two carbon atoms of the chain is bonded to two same or different lower alkyl groups each containing 1 to 12 carbon atoms or to an alicyclic group containing 4 to 9 carbon atoms which groups sterically hinder the amine and exposing the formed article to a sterilising dose of gamma-irradiation from a cobalt-60 source.

9. A method according to claim 8 wherein the sterilising dose of irradiation is from 2.5 to 5.0 Mrad.

**Patentansprüche**

1. Gegenstand für medizinische Zwecke, der aus einem Spritzenbestandteil, einem Katheter, einer Kanüle oder einer Protheseneinrichtung besteht, durch Gammabestrahlung aus einer Kobalt 60-Quelle sterilisierbar ist und aus einer Masse hergestellt wurde, die ein kristallines Polymer eines aliphatischen $\alpha$-Olefins enthält, dadurch gekennzeichnet, daß die Masse 0,01 bis 2 Gew.-%, auf das Gewicht des Polymers bezogen, eines behinderten Amins oder von dessen Salz, N-Oxid, N-Hydroxid oder N-Nitroxid enthält, wobei der Aminstickstoff in einer Kohlenstoff-Stickstoff-Kohlenstoff-Kette, die einen Teil eines nichtaromatischen heterocyclischen Ringes bildet, enthalten ist und wobei jedes der zwei Kohlenstoffatome der Kette an zwei gleiche oder verschiedene niedere Alkylgruppen mit jeweils 1 bis 12 Kohlenstoffatomen, die das Amin sterisch behindern, oder an eine alicyclische Gruppe mit 4 bis 9 Kohlenstoffatom, die das Amin sterisch behindert, gebunden ist.

2. Gegenstand nach Anspruch 1, bei dem das behinderte Amin einen heterocyclischen Sechsring enthält.

3. Gegenstand nach Anspruch 2, bei dem das behinderte Amin von einem Träger, der ein Dicarboxylat enthält, getragen wird.

4. Gegenstand nach Anspruch 2, bei dem das behinderte Amin von einem Träger, der einen Triazinring enthält, getragen wird.

5. Gegenstand nach Anspruch 2, bei dem das behinderte Amin von einem Träger, der ein Diketon enthält, getragen wird.

6. Gegenstand für medizinische Zwecke, der aus einem Spritzenbestandteil, einem Katheter, einer Kanüle oder einer Protheseneinrichtung besteht, durch Gammabestrahlung aus einer Kobalt 60-Quelle sterilisiert wurde und aus einer Masse hergestellt wurde, die ein kristallines Polymer eines aliphatischen $\alpha$-Olefins enthält und 0,01 bis 2 Gew.-%, auf das Gewicht des Polymers bezogen, eines behinderten Amins oder von dessen Salz, N-Oxid, N-Hydroxid oder N-Nitroxid enthält, wobei der Aminstickstoff in

einer Kohlenstoff-Stickstoff-Kohlenstoff-Kette, die einen Teil eines nichtaromatischen heterocyclischen Ringes bildet, enthalten ist und wobei jedes der zwei Kohlenstoffatome der Kette an zwei gleiche oder verschiedene niedere Alkylgruppen mit jeweils 1 bis 12 Kohlenstoffatomen, die das Amin sterisch behindern, oder an eine alicyclische Gruppe mit 4 bis 9 Kohlenstoffatomen, die das Amin sterisch behindert, gebunden ist.

7. Gegenstand nach Anspruch 6, der zwei Wochen nach der Bestrahlung einen Verfärbungsfaktor, der desser als 0,750 ist, aufweist und in einem Ofen an der Luft bei 150°C eine Haltbarkeit von mindestens 6 Tagen hat, wobei der Gegenstand aus einer Kobalt 60-Quelle eine Strahlungsdosis von 5,0 Mrd (50 kJ/kg) empfangen hat.

8. Verfahren zum Formen eines sterilisierten Gegenstandes für medizinische Zwecke, der aus einem Spritzenbestandteil, einem Katheter, einer Kanüle oder einer Protheseneinrichtung besteht, dadurch gekennzeichnet, daß der Gegenstand aus einer Masse geformt wird, die 0,01 bis 2 Gew.-%, auf das Gewicht des Polymers bezogen, eines behinderten Amins oder von dessen Salz, N-Oxid, N-Hydroxid oder N-Nitroxid enthält, wobei der Aminstickstoff in einer Kohlenstoff-Stickstoff-Kohlenstoff-Kette, die einen Teil eines nichtaromatischen heterocyclischen Ringes bildet, enthalten ist und wobei jedes der zwei Kohlenstoffatome der Kette an zwei gleiche oder verschiedene niedere Alkylgruppen mit jeweils 1 bis 12 Kohlenstoffatomen, die das Amin sterisch behindern, oder an eine alicyclische Gruppe mit 4 bis 9 Kohlenstoffatomen, die das Amin sterisch behindert, gebunden ist, und daß der geformte Gegenstand einer sterilisierenden Dosis von Gammabestrahlung aus einer Kobalt 60-Quelle ausgesetzt wird.

9. Verfahren nach Anspruch 8, bei dem die sterilisierende Bestrahlungsdosis 2,5 bis 5,0 Mrd (25 bis 50 kJ/kg) beträgt.

## Revendications

1. Objet ou article de matériel médical constitué par un élément de seringue, un cathéter, une canule ou un dispositif prothétique, cet objet ou article étant stérilisable par irradiation par des rayons gamma à partir d'une source de cobalt 60 et étant constitué à partir d'une composition renfermant un polymère cristallin d'une alpha-oléfine aliphatique, caractérisé en ce que la composition renferme de 0,01 à 2% en poids du polymère d'une amine à empêchement stérique ou de son sel, de son N-oxyde, de son N-hydroxyde ou de son N-nitroxyde, où l'azote de l'amine est contenu dans une chaîne carbone-azote-carbone qui fait partie d'un hétérocycle non aromatique, et où chacun des 2 atomes de carbone de la chaîne est lié à deux groupes alcoyle inférieur identiques ou différents contenant chacun de 1 à 12 atomes de carbone ou à un groupe alicyclique contenant de 4 à 9 atomes de carbone, ces groupes provoquant un empêchement stérique de l'amine.

2. Objet ou article suivant la revendication 1, caractérisé en ce que l'amine à empêchement stérique comprend un hétérocycle à 6 chaînons.

3. Objet ou article suivant la revendication 2, caractérisé en ce que l'amine à empêchement stérique est portée par une fraction porteuse constituée par un dicarboxylate.

4. Objet ou article suivant la revendication 2, caractérisé en ce que l'amine à empêchement stérique est portée par une fraction porteuse constituée par un cycle triazine.

5. Objet ou article suivant la revendication 2, caractérisé en ce que l'amine à empêchement stérique est portée par une fraction porteuse constituée par une dicétone.

6. Objet ou article de matériel médical constitué par un élément de seringue, an cathéther, une canule ou und dispositif prothétique stérilisé par irradiation par des rayons gamma à partir d'une source de cobalt 60 et réalisé à partir d'une composition renfermant un polymère cristallin d'une alpha-oléfine aliphatique, cette composition renfermant de 0,01 à 2% en poids du polymère d'une amine à empêchement stérique ou de son sel, de son N-oxyde, de son N-hydroxyde ou de son N-nitroxyde, où l'azote de l'amine est contenu dans une chaîne carbone-azote-carbone qui fait partie d'un hétérocycle non-aromatique et où chacun des deux atomes de carbon de la chaîne est lié à deux groupes alcoyle inférieur identiques ou différents contenant chacun de 1 à 12 atomes de carbone ou à un groupe alicyclique contenant de 4 à 9 atomes de carbone, ces groupes provoquant un empêchement stérique de l'amine.

7. Objet ou article suivant la revendication 6, ayant un facteur de décoloration meilleur que 0,750 deux semaines après l'irradiation et une longévité au four dans l'air à 150°C d'au moins 6 jours, cet objet ou article ayant reçu une dose de radiation de 5,0 Mrads à partir d'une source de cobalt 60.

8. Procédé pour la formation d'un objet ou article stérilisé de matériel médical constitué par un élément de seringue, un cathéter, une canule ou un dispositif prothétique, caractérisé en ce qu'on réalise cet objet ou article à partir d'une composition renfermant de 0,01 à 2% en poids, sur la base du polymère, d'une amine à empêchement stérique ou de son sel, de son N oxyde, de son N-hydroxyde et de son N-nitroxyde, où l'azote de l'amine est contenu dans une chaîne carbone-azote-carbone qui fait partie d'un hétérocycle non-aromatique et où chacun des deux atomes de carbone de la chaîne est relié à des groupes alcoyle inférieur identiques ou différents contenant chacun de 1 à 12 atomes de carbone ou à un groupe alicyclique contenant de 4 à 9 atomes de carbone, ces groupes provoquant un

empêchement stérique de l'amine, et à exposer l'objet ou article formé à une dose stérilisante d'irradiation par des rayons gamma à partir d'une source de cobalt 60.

9. Procédé suivant la revendication 8, caractérisé en ce que la dose stérilisante d'irradiation est comprise entre 2,5 et 5,0 Mrads.